# EUROPEAN PATENT SPECIFICATION

(11) **EP 1 329 009 B1**
(45) Date of publication and mention of the grant of the patent: **22.03.2006**
(21) Application number: 01963292.6
(22) Date of filing: 27.08.2001
(51) Int. Cl.: H02G 7/20

(54) **POLE TOP SUPPORT FOR AERIAL ELECTRIC POWER LINES**
PFOSTENSTÜTZKOPF FÜR ELEKTRISCHE FREILEITUNGEN
SUPPORT DE TETE DE POTEAU POUR LIGNES AERIENNES DE TRANSPORT D'ENERGIE ELECTRIQUE

(30) Priority: 28.08.2000 IT TO000822
(43) Date of publication of application: 23.07.2003
(73) Proprietor: Conveytech S.p.A., 10138 Torino (IT)
(72) Inventor: BOSCHETTI, Mario, I-11020 Donnas (IT); BOSCHETTI, Claudio, I-11020 Donnas (IT)
(74) Representative: Dini, Roberto
(86) International application number: PCT/IB2001/001546
(87) International publication number: WO 2002/019493

(56) References cited:
- FR-A- 1 129 240
- US-A- 3 603 717
- US-A- 3 803 345

## Description

The present invention relates to a top pole support for aerial electric power lines, in particular with suspended wires, in which said aerial electric power lines comprise at least three wires and said top pole support comprise insulating means, in particular suspended insulating means, to support said wires, according to claim 1. Preferred embodiments of the invention are set out in the dependent claims.

Aerial electric power lines for medium and high voltage, as known, are supported through suitable poles that comprise a variety of top pole supports, i.e. supports to sustain the wires of the aerial electric power lines.

Said top pole supports are usually built through a straight structure of bars, like cantilever or trellis, i.e. formed by several pieces for assembly, to which structure insulators or chain of insulators are connected to support the current wires.

The medium voltage lines in the past used to mount on the top pole support stiff insulators, that were less suitable to resist to stress from a mechanical and electrical point of view that corresponding suspended insulator, i.e. hanging from the structure of the top pole support.

Therefore, faced with the need of converting lines with stiff insulators into lines with suspended insulators, it is necessary to substitute the top pole support, that should have the following features:
- it has to support the wires at a height such that the wires are not very low along the span;
- It must have an open structure in order to nest the central wire without having to cut and joint again such wire;

US 3,803,345 discloses a crossarm for a utility pole possessing a curvilinear configuration and having looped supports distributed along the curvilinear configuration at different elevations for supporting ceramic insulators over which high voltage conductors are strung. The crossarm is made from plastic extrusions and moldings.

US 3,603,717 discloses a crossarm assembly with an arcuate crossann and a pair of legs for supporting the arcuate crossarm on a pole. Channel-shaped brackets are used to secure the arcuate crossarm to each leg while a dowel pin is inserted within mating recesses of the crossarm and the support legs in order to position the crossarm and to resist the loading of the crossarm.

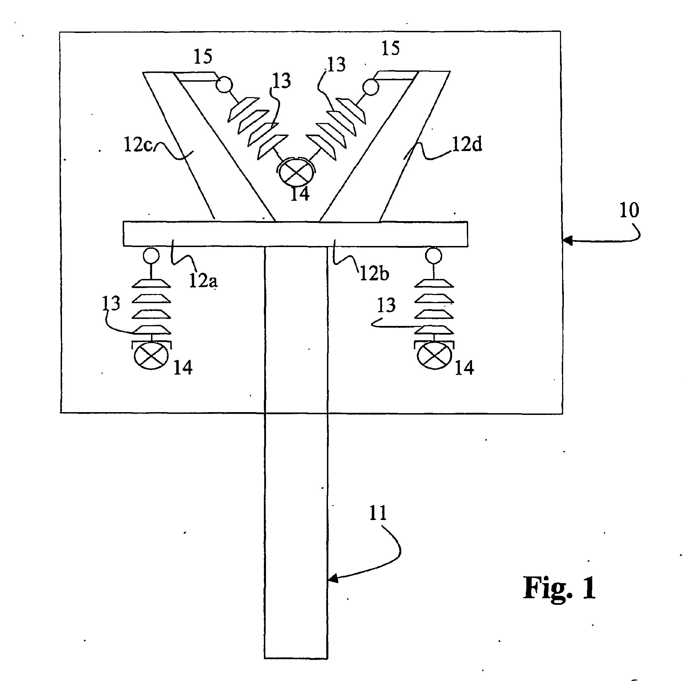

In figure 1 a top pole support 10 according to the prior art is shown, that is fit on the top of a pole 11, and is composed by four arms 12a, 12b, 12c, 12d, i.e., the above mentioned straight bars, the two arms 12a and 12b perpendicularly protruding from the pole 11, in alternatively opposite directions, while arms 12c and 12d protrude in an oblique way above the pole 11, in a symmetrical position with respect to the axis of said pole 11. Each of said four arms 12 shows, assembled on the distal extremity with respect to pole 11, a chain of suspended insulators 13, to which corresponding wires 14 of the electrical aerial line are hooked. Arms 12a and 12b show a chain of insulators 13 each, that extends downward vertically and carries a wire 14, Arms 12c and 12d show little beams 15 on which the corresponding chains ofinsulators 13, that support together the third wire 14, are connected.

Support know in the prior art, as the one shown in figure 1, show several drawbacks associated to great weight and cost of the structure, that is further disadvantageously constituted by several pieces to be assembled on the pole.

Moreover the the "V" shaped chains associated to the arms 12c and 12d are too heavy and they do not stretch completely when in use with the lighter wires.

Another drawback of the known supports resides in the necessity to distance enough with respect to each other the insulators chains that are at the same height, in order to comply with the specifications regarding the distance between wires in the span in presence of wind.

In the same way has to be avoided the vertical alignment of the insulator chain positions, since the formation during wintertime of icicles on the wires and the subsequent detachment provokes whip lash reactions that could shortcircuit two wires being one above the other.

The operation of substituting the top pole support can be performed with the line in off state. It is often more convenient to operate with the voltage applied, in order not to interrupt the service for the users. The operation of substitution of the top pole support requires a top pole support with a structure that allows the assembly by simple and safe operations, that can be performed by the operators standing always at determined safety distances from the wires.

The present invention has for aim to solve the abovementioned drawbacks and to indicate a top pole support for aerial electric power lines, in particular with suspended wires, having an improved construction and more efficient with respect to the known solutions.

Under this frame, the main object of the present invention is to indicate a top pole support for aerial electric power lines, in particular with suspended wires, apt to simplify its own installation and substitution.

A further object of the present invention is to indicate a top pole support for aerial electric power lines, in particular with suspended wires, that does not require peculiar assembly operation to be performed directly on the pole top.

A further object of the present invention is to indicate a top pole support for aerial electric power lines, in particular with suspended wires, that allows for an optimal spacing of the wires.

A further object of the present invention is to indicate a top pole support for aerial electric power lines, in particular with suspended wires, that supports the wires at an height sufficient not to sensibly lower the wires along the span.

A further object of the present invention is to indicate a top pole support for aerial electric power lines, in particular with suspended wires, that has an open structure in order to receive the central wire without the need of cutting and the jointing said wire.

In order to achieve such aims, it is the object of the present invention to provide a top pole support for aerial electric power lines, in particular with suspended wires, incorporating the features of the annexed claims, which form an integral part of the description herein.

Further objects, features and advantages of the present invention will become apparent from the following detailed description and annexed drawings, which are supplied by way of non limiting example, wherein:
- figure 1 shows a basic diagram of provide a top pole support for aerial electric power lines, in particular with suspended wires, according to the prior art;
- figure 2 shows a basic diagram of provide a top pole support for aerial electric power lines, in particular with suspended wires, according to the invention;
- figures 3 a and 3b show two views of a variant to the top pole support shown in figure 2.

The inventive idea consists basically in carrying out a top pole support formed by a sole element, that is curvilinear, in order to substantially pass by the points where is desired that are hooked the insulating means sustaining the wires, that usually lie on the vertexes of a triangle that is placed in a way that avoids horizontal or vertical alignment of the wires.

A further feature of the invention is that said curvilinear element can develop in a curve that does not lie in a sole plane.

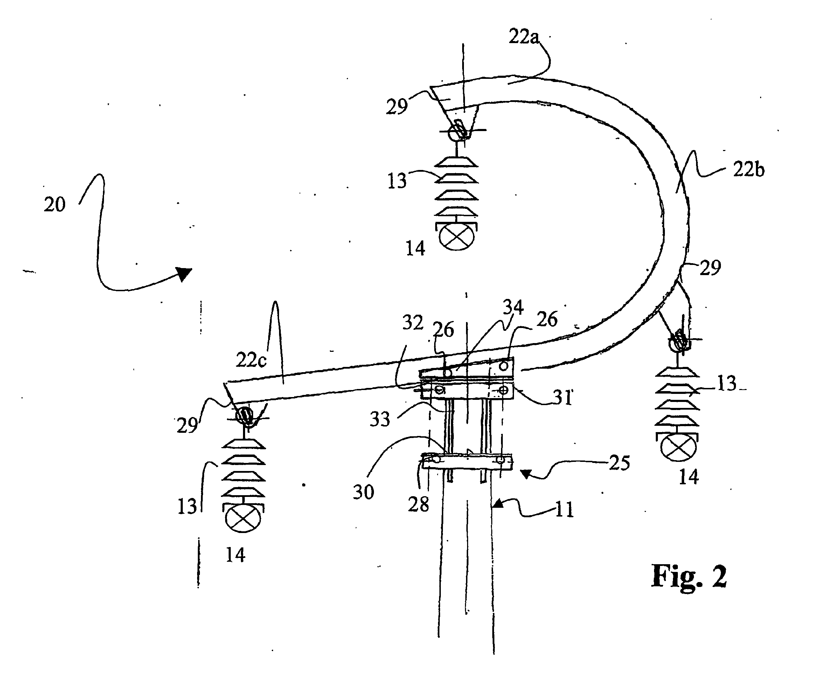

In figure 2 a top pole support 20 for aerial electric power lines according to the invention is shown. Said support 20 is shaped in a sole piece, in example a high resistance Mannesmann tube, that makes a continuous curve, substantially in a shape of a upside down "C", where a upper arm 22, a bend 22b and a lower arm 22c can be identified.

Said lower arm 22c is longer than the upper arm 22a, and also slightly downwards curved. On the extremes of the upper arm 22a and of the lower arm 22c to connections 29 are secured respective chain of suspended insulators 13 that, on their distal extreme bear the wires 14. On an external point of tangency to the bend 22b in the same way is fixed a connection 29 for a chain of insulators 13 and the corresponding third wire 14.

Bend 22b is designed in order to have a shape such to allow to the chain of insulators 13 of the upper arm 22a to swing in presence of wind, as prescribed by safety rules, without reaching during the swing said bend 22b, i.e. mantaining the correct distance among parts under voltage and grounded parts.

The top pole support 20 according to the invention is fastened to a metallic crate 25 through screw and bolts couplings, that work in four suitable bore 27, made on the middle part of the lower arm 22c. The crate 25 comprises substantially two lower clamps 30, only one of which is visible in figure 2, coupled together through horizontal screws 28, and two upper clamps 31, coupled together through horizontal screws 32. The upper clamps 31 and the lower clamps 30 are then coupled together through four welded vertical tubes 33. The upper clamps 31 carry on their upper part wings 34, that are fastened to the top pole support 20 through the screw and bolt couplings 26.

When it is necessary to install the support 20 according to the invention is possible to use a little derrick, that can be also fastened to the horizontal screws 28 and 32 of the crate 22, to lift the whole support 20, already fastened to the crate 25, and let it reach the pole top 11, where it is leaned, fitted and fastened with a minimal effort by an operator by tightening the screws 28 and 32 through suitable bolts.

The operator, during the execution of said operation, operates substantially at the height of the crate 25, remaining conveniently far from the wires 14, that, as mentioned, can be under voltage and that, consequently, in this case, during the substitution operation are moved away to a safety distance from the top of the pole 11 through insulated spikes.

Since the operator has to operate only at the height of the crate 25, without the need of climbing higher for fastening further elements, it is a lot easier for the operator to maintain the control of the safety distance from the parts under voltage, both with respect to his body and with respect to the tools that he uses.

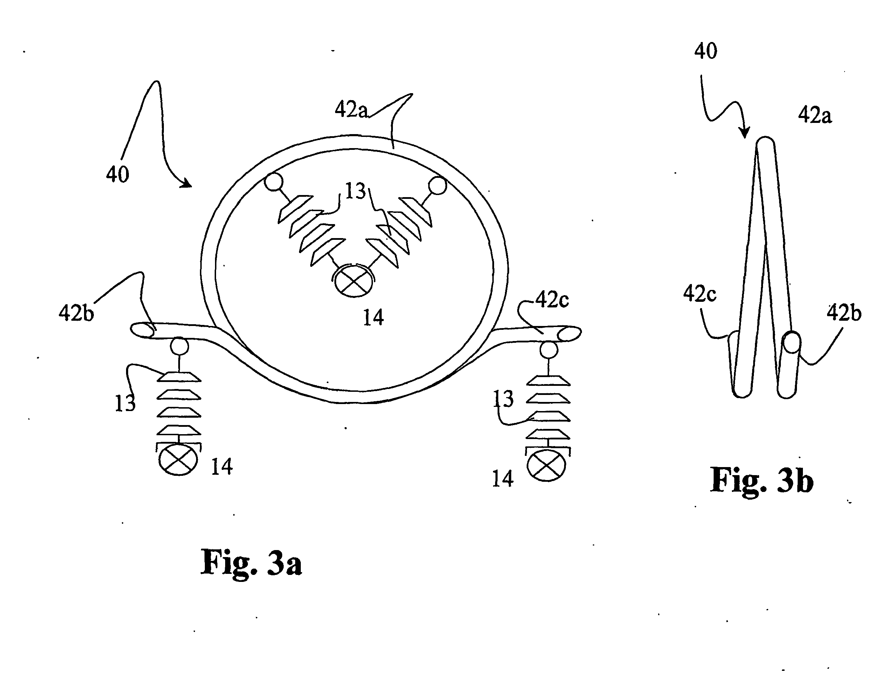

In figure 3a and 3b a frontal view (fig. 3a) and a lateral view (fig. 3b) of a pole top support 40 for aerial electrical lines, variant to the pole top support 20 of figure 2. The pole top support 40 is spirally shaped in this case, with a bend 42a and two arms 42b and 42c that are symmetrical in the plane of the spiral and are raised in a suitable way with respect to the point of support of the pole top support 40 in order to raise the chains of insulators 13.

The curve described by said top pole support 40, as can be better seen in figure 3b, where for simplicity's sake only the spiral shaped tube with the chains of insulators 13 is shown, does not lie in a single plane, but on the contrary the curve develops in space in a way that allows for insertion of the central wire without the need of cutting and then jointing it.

Said top pole support 40, in an analogous way to the top pole support 10 shown in figure 1, makes use of to chains of insulator 13 on the bend 42a, that support together the third wire 14.

Of course, the top pole support 40 can also use a sole chain 13 on the bend 42a to suspend the central wire 14.

The spiral shape of the top pole support 40 gives to an intrinsically simple and light structure a remarkable mechanical resistance against stresses exerted in parallel with the line direction.

From the above description the features of the present invention as well as the relevant advantages thereof are clear.

The top pole support for aerial electric power lines, in particular with suspended wires according to the invention, advantageously is obtained in a single piece that can be assembled before the assembly the pole and can be easily fastened, making very simple, safe and costless the installation, substitution and maintenance operations, in particular operation performed with the voltage applied.

Advantageously the "C" shape of the top pole support for aerial electric power lines, in particular with suspended wires according to the invention allows to avoid horizontal and vertical alignments of the chains of suspended insulators, although only one element is used.

Further, advantageously, the top pole support for aerial electric power lines, in particular with suspended wires according to the invention ensures an higher safety for the operators during the installation and substitution, since its weight is fully supported by a lifting device as the derrick, so that the operator has only to concentrate on the fastening on the top of the pole.

Further the circular section of the tube with which the top pole support according to the invention is carried out, offers a better structural efficiency, in particular with respect to supports with cantilever straight bars, fastened through bolts. Further, advantageously, said structure of the top pole support, according to the invention is elastic, giving an higher mechanical resistance to dynamical stresses.

It is obvious that many changes are possible for the man skilled in the art top pole support for aerial electric power lines, in particular with suspended wires according to the invention described above by way of example, without departing from the novelty spirit of the innovative idea, and it is also clear that in practical actuation of the invention the components may often differ in form and size from the ones described and be replaced with technical equivalent elements.

In particular, the shape of the curve described by the support could be different. In other word, it is apparent that it is possible to change the shape of the support without departing from the inventive concept of having a support obtained through a curvilinear continuous element that passes by the point where the chains of insulators that hold the wires are.

As already described, it will be possible that said curve traced by the top pole support according to the invention, by way of example, does not lies in one plane only, but it will spread in space according to the structural necessities and the placement of the chains of insulators.

The top pole support according to the invention could be carried out not only, through tubes, but also through box structures or section bars.

It will be also possible, in order to match the necessities of geometry with that of safety distances, to adapt the shape of the connections and of the chains of insulators.

The top pole support can be advantageously obtained, all or in part, through insulating material, in order to increase insulation and, eventually, decrease the insulation of the chains that hold the wires.

## Claims

1. Top pole support for aerial electric power lines, suitable for being fastened on the top of a pole, in which said aerial electric power lines comprise at least three wires (14) and said top pole support (10; 20; 40) comprises corresponding insulating means (13) to hold said wires (14), wherein said top pole support (20; 40) has a shape that traces a continuous curve substantially passing by the point in which the insulating means (13) are, said curve being a continuous open curve, and wherein said top pole support (20;40) defines an open structure when fastened to the top of the pole in order not to interrupt the service for users when receiving a central wire, **characterized in that** said insulating means (13) and said at least three wires (14) are suspended, and **in that** said top pole support (10; 20; 40) is fastened to the upper end surface of the top of the pole.

2. Top pole support for aerial electric power lines according to claim 1, **characterized in that** said top pole support (20; 40) is fastened through a crate (25) to the pole (11).

3. Top pole support for aerial electric power lines according to claim 1 or 2, **characterized in that** said continuous open curve comprises a bend (22b), a lower arm (22c) and an upper arm (22a).

4. Top pole support for aerial electric power lines according to claim 3 **characterized in that** the insulating means (13), in particular suspended insulators, are connected to the extremity of the lower arm (22c), of the upper arm (22a) and on the external part of the bend (22b).

5. Top pole support for aerial electric power lines according to at least one of the preceding claims, **characterized in that** said top pole support (20) is carried out through a tubular element.

6. Top pole support for aerial electric power lines according to at least claim 2, **characterized in that** said top pole support (40) traces an open continue curve that does not lie in only one plane.

7. Top pole support for aerial electric power lines according to at least claim 2, **characterized in that** said top pole support (40) is spiral shaped with a bent part (42a) and two arms (42b, 42c) to the extremities substantially symmetrical in the plane of the spiral.

## Patentansprüche

1. Leitungsmastträgerspitze für elektrische Freileitungen, die geeignet ist, an der Spitze eines Leitungsmastes befestigt zu werden, bei der die elektrischen Freileitungen mindestens drei Leitungen (14) umfassen und die Leitungsmastträgerspitze (10, 20, 40) entsprechende Isolationsmittel (13) zum Halten der Leitungen (14) umfasst, wobei die Leitungsmastträgerspitze (20, 40) eine Form besitzt, die einer kontinuierlichen Kurve folgt, die an den Punkten, an denen sich die Isolationsmittel (13) befinden, vorbeiführt, wobei die Kurve eine kontinuierliche offene Kurve ist und wobei die Leitungsmastträgerspitze (20, 40) eine offene Struktur definiert, wenn sie an der Spitze des Leitungsmastes befestigt ist, um den Betrieb für Benutzer bei der Aufnahme einer zentralen Leitung nicht zu unterbrechen,
**dadurch gekennzeichnet, dass** die Isolationsmittel (13) und die mindestens drei Leitungen (14) aufgehängt sind und dass die Leitungsmastträgerspitze (10, 20, 40) an der oberen Endoberfläche der Spitze des Leitungsmastes befestigt ist.

2. Leitungsmastträgerspitze für elektrische Freileitungen nach Anspruch 1,
**dadurch gekennzeichnet, dass** die Leitungsmastträgerspitze (20, 40) über einen Rahmen (25) an dem Mast (11) befestigt ist.

3. Leitungsmastträgerspitze für elektrische Freileitungen nach Anspruch 1 oder 2,
**dadurch gekennzeichnet, dass** die kontinuierliche offene Kurve eine Biegung (22b), einen unteren Arm (22c) und einen oberen Arm (22a) aufweist.

4. Leitungsmastträgerspitze für elektrische Freileitungen nach Anspruch 3,
**dadurch gekennzeichnet, dass** die Isolationsmittel (13), insbesondere aufgehängte Isolatoren, mit dem äußeren Ende des unteren Arms (22c), dem äußeren Ende des oberen Arms (22a) und dem äußeren Bereich der Biegung (22b) verbunden sind.

5. Leitungsmastträgerspitze für elektrische Freileitungen nach einem der vorangehenden Ansprüche,
**dadurch gekennzeichnet, dass** die Leitungsmastträgerspitze (20) durch ein röhrenförmiges Element ausgeführt ist.

6. Leitungsmastträgerspitze für elektrische Freileitungen nach zumindest Anspruch 2,
**dadurch gekennzeichnet, dass** die Leitungsmastträgerspitze (40) einer offenen kontinuierlichen Kurve folgt, die in nicht nur einer Ebene liegt.

7. Leitungsmastträgerspitze für elektrische Freileitungen nach zumindest Anspruch 2,
**dadurch gekennzeichnet, dass** die Leitungsmastträgerspitze (40) spiralförmig ist mit einem gebogenen Teil (42a) und zwei Armen (42b, 42c), die sich zu ihren äußeren Enden im wesentlichen symmetrisch in der Ebene der Spirale erstrecken.

## Revendications

1. Support de tête de poteau pour lignes aériennes de transport d'énergie électrique, approprié pour être fixé sur la tête d'un poteau, dans lequel lesdites lignes aériennes de transport d'énergie électrique comprennent au moins trois câbles (14) et ledit support de tête de poteau (10 ; 20 ; 40) comprend des moyens isolants correspondants (13) pour maintenir lesdits câbles (14), dans lequel ledit support de tête de poteau (20 ; 40) a une forme qui trace une courbe continue qui passe sensiblement par le point auquel se trouvent les moyens isolants (13), ladite courbe étant une courbe continue ouverte, et dans lequel ledit support de tête de poteau (20 ; 40) définit une structure ouverte lorsqu'il est fixé sur la tête du poteau, afin de ne pas interrompre le service aux usagers lorsqu'il reçoit un câble central, **caractérisé en ce que** lesdits moyens isolants (13) et lesdits au moins trois câbles (14) sont suspendus, et **en ce que** ledit support de tête de poteau (10 ; 20 ; 40) est fixé sur la surface d'extrémité supérieure de la tête du poteau.

2. Support de tête de poteau pour lignes aériennes de transport d'énergie électrique selon la revendication 1, **caractérisé en que** ledit support de tête de poteau (20 ; 40) est fixé par l'intermédiaire d'un cadre (25) au poteau (11).

3. Support de tête de poteau pour lignes aériennes de transport d'énergie électrique selon la revendication 1 ou 2, **caractérisé en que** ladite courbe ouverte continue comprend un arrondi (22b), un bras inférieur (22c) et un bras supérieur (22a).

4. Support de tête de poteau pour lignes aériennes de transport d'énergie électrique selon la revendication 3, **caractérisé en que** les moyens isolants (13), en particulier des isolateurs suspendus, sont connectés à l'extrémité du bras inférieur (22c), du bras supérieur (22a) et sur la partie extérieure de l'arrondi (22b).

5. Support de tête de poteau pour lignes aériennes de transport d'énergie électrique selon au moins l'une des revendications précédentes,
**caractérisé en ce que** ledit support de tête de poteau (20) est réalisé par l'intermédiaire d'un élément tubulaire.

6. Support de tête de poteau pour lignes aériennes de transport d'énergie électrique selon au moins la revendication 2, **caractérisé en ce que** ledit support de tête de poteau (40) trace une courbe ouverte continue qui ne se trouve pas dans un plan unique.

7. Support de tête de poteau pour lignes aériennes de transport d'énergie électrique selon au moins la revendication 2, **caractérisé en ce que** ledit support de tête de poteau (40) a la forme d'une spirale avec une partie arrondie (42a) et deux bras (42b, 42c) jusqu'aux extrémités sensiblement symétriques dans le plan de la spirale.
